# EUROPEAN PATENT SPECIFICATION

(11) **EP 3 318 191 B1**
(45) Date of publication and mention of the grant of the patent: **03.03.2021**
(21) Application number: 16750874.6
(22) Date of filing: 29.06.2016
(51) Int. Cl.: A61B 5/15, A61M 5/142, A61M 5/32

(54) **SYSTEM FOR COLLECTING BLOOD SAMPLES FROM A PATIENT, AND CORRESPONDING SAMPLE COLLECTION DEVICE AND PUMPS FOR EXTRACTING BLOOD FROM A PATIENT**
SYSTEM ZUM SAMMELN VON BLUTPROBEN VON EINEM PATIENTEN UND ENTSPRECHENDE PROBENSAMMELVORRICHTUNG UND PUMPEN ZUR BLUTENTNAHME VON EINEM PATIENTEN
SYSTÈME DE PRÉLÈVEMENT D'ÉCHANTILLONS DE SANG D'UN PATIENT, ET DISPOSITIF DE PRÉLÈVEMENT D'ÉCHANTILLONS ET POMPES CORRESPONDANTES POUR L'EXTRACTION DU SANG D'UN PATIENT

(30) Priority: 02.07.2015 EP 15382352
(43) Date of publication of application: 09.05.2018
(73) Proprietor: Cliniclab S.L., 08140 Caldes de Montbui (ES)
(72) Inventor: XALABARDER MIRAMANDA, Ferran, 08140 Caldes de Montbui (Barcelona) (ES)
(74) Representative: Gallego Jiménez, José Fernando
(86) International application number: PCT/ES2016/070484
(87) International publication number: WO 2017/001715

(56) References cited:
- EP-A2- 0 747 087
- WO-A1-02/058556
- WO-A1-2013/164358
- DE-U1- 29 707 813
- FR-A1- 2 647 351
- JP-A- H10 179 737
- US-A- 4 008 717
- US-A- 4 425 119
- US-A- 5 046 509
- US-A- 5 201 716

## Description

### Field of the invention

The invention relates to a system for taking blood samples from a patient.

The invention also relates to a sampling device and to a pump for extracting blood from a patient.

### State of the art

Since prior to the decade of the sixties, the evolution of clinical analysis laboratories for the diagnosis and control of the treatments of diseases in persons and animals has followed such a spectacular advance that it is today one of the fields of the health care sector that is deemed to be absolutely necessary for the present level of social life. The technical development of the clinical analysis laboratories has prospered daily vertiginously both in the automation of the analytical techniques and in the appliances, as well as in the knowledge of new techniques for carrying out the analyses which are basically performed with biological samples from the patient or individual of blood, urine, faeces, hair, nails, skin, etc. Of all these possible analytical samples, the most used is the blood sample, both of venous and of arterial or capillary origin. In each analytical case, the most appropriate sample will be used. The number of blood samples increases annually, being currently in Spain around 120 million extractions per year.

Of the different types of blood samples used for performing the analyses, the most frequent are venous blood samples.

Practically all of the methods currently used for blood extraction are over 30 years old, without having evolved or been notably improved. This slight evolution of venous blood extraction systems has occurred with respect to the use of new materials (for example, glass has given way to plastics), to new systems for manufacturing the components for making the venous blood extraction, but the extraction methods or systems are practically the same.

### a) Among the blood sample extraction systems the following can be cited:

1. The oldest system consists of making a wound in a capillary area and collecting the blood in a micro or capillary tube, or puncturing a vein or artery with a hypodermic needle so that under its own pressure it flows out of the skin an d the sample is collected directly in a container, a test tube, on a slide, etc.
2. To obviate the slowness of the above system and obtain a larger volume of blood more quickly, there is used the system of a hypodermic needle together with a syringe, to aspirate with the syringe plunger the volume of blood more quickly and in a larger amount in the shortest possible time to avoid coagulation thereof. This method, still widely used currently (in developed countries, UK, USA, France, Spain, etc., it represents approximately 30% of blood extractions) has improved in the quality of the hypodermic needle and in the syringes with high quality and precision manufacturing processes. Said system has good applications for the ease of handling thereof and is extremely useful for difficult veins. It is generally used for its cheapness and the high quality of the analytical blood sample, provided that the operator making the extraction acts correctly. In this procedure, the operator aspirating the blood from inside of the vein with the syringe can create an excessive "vacuum" and in such case can cause hemolysis of the blood, i.e. the breakage of the red blood cells, and mixing with the serum or plasma thereof. This problem can be extremely serious, causing interference or false results on obtaining erroneous samples when performing the analytical techniques. Another drawback of the system is that the operator extracting the sample has to meter the precise volume of blood to be mixed with an anticoagulant or reagent, which may cause a bad metering of the sample to produce an incorrect analytical blood sample for certain analyses. A further drawback of the system is that currently there are required blood samples contained in different tube formats (from 3 to 8) with different anticoagulants or reagents for use in different apparatus r analytical techniques. Namely, a large amount of samples are required from the same patient when extracting, so that thereafter they may be distributed around the different laboratory departments, without having to transfer the blood sample from one tune to another, thereby avoiding possible contagion of diseases which the freshly extracted blood sample may transmit, by dropping, spilling or breakage of the tubes, or to be able to send them to other laboratories more specialized in less frequent analytical techniques. This obliges the use of large volume syringes (20 ml or more) making the extraction more expensive, as well as metering the distributing the extracted blood into a plurality of blood sample containers or tubes. This process is always conditioned to the more or less correct handling by the operator making the extraction. This method has been improved with the application of the stopper for laboratory tubes marketed under the name of TAPVAL®.
3. To eliminate some of the above mentioned drawbacks, a new process was developed which uses a container in which a vacuum has been created prior to use thereof. This procedure consists of extracting the blood with a special hypodermic needle with one end of which the vein is punctured and which at the other end thereof has another needle with a resilient, rubber type hood which acts as a valve and allows closing or opening when puncturing with the needle the stopper of the tube under vacuum. When connecting the vacuum with the interior of the vein through the other end of the needle, a certain amount of blood is aspirated depending on the volume of vacuum inside the container. This "by vacuum" system has many drawbacks, in practice important, such as are the large amount of hemolysed samples, the incorrect metering of the blood volume in many tubes, the difficult practice of puncturing the vein causing many bruises for the patient for excessive breakage of the vein (perforation of both sides of the vein), the need for using special needles, the difficulty of disposing of them and their high cost at the time of the extraction. Subsequently, in the laboratory, having to unstopper the tube for using the content causes serious, hard to solve, problems such as are possible contamination by aerosols, spilling, breakage of the glass tube mouth, etc. Although there are currently vacuum extraction systems with not so fragile plastic tubes, these have other technical problems, such as the evaporation or desiccation of the water-based reagents which cause problems of incorrect dilution, with a significant alteration of the results of the analyses. Another drawback is that small volume container tubes (1 ml, 0.5 ml or 0.1 ml) cannot be used. It should be borne in mind that, currently, owing to the new technologies and the new autoanalyzers for pediatric techniques, several small samples in different tubes are required, it being necessary to perform capillary punctures, with all their serious drawbacks.
4. Another system, which may be considered to be a mixture of the two above-mentioned methods (the syringe and the vacuum tube), is the one consisting of using a special needle like the one sued in the vacuum container, which is attached to a tube having a special stopper, with a membrane of a resilient, rubber type, material which admits being perforated by the special needle having a rubber sheath which acts as a seal when interchanging the tubes for filling thereof by means of a plunger with which there is aspired the volume of blood necessary for each tube-syringe, partly eliminating the problems of the large volume syringe and the fixed vacuum of the aforementioned vacuum tubes.

All these methods present the same or similar practical problems due to the handling by the health care operator. Depending on the skills of the latter, the extraction will be correct or not, since presently the personnel making the extractions have limited technical and health knowledge and scarce technical relationship with the laboratory performing the analyses.

Another serious drawback is the unstoppering of the tube for extracting the blood sample from the interior thereof for performing the pertinent analyses.

In the document "Errors in clinical laboratories or errors in laboratory medicine?" M. Plebany, Clin. Chem. Lab. Med. 2006; 44(6); 750-759, Walter de Gruyter, Berlin, New York, DOI 10.1515/CCLM.2006.125 various errors which may be made while taking blood samples are commented upon.

The most important risk of these blood extraction systems is that the needle inserted in the vein has to be disposed of and this disposal is usually effected by using the other hand and it is then when there is a great danger of an occupational accident, by being pricked with a needle which may be contaminated. During the year 2000 there were in Spain 3,514 cases of accidental exposure to blood and other body fluids by pricks or cuts among the health care personnel, generally in nursing personnel who suffer 87% of these accidents. In 5% of these cases there is a risk of contagion by HIV and in 14% by Hepatitis "C". Thus the rate of infection by HIV is estimated to be 3 cases for every 300 pricks, it being generally unknown whether the patient had this type of infection or not The avoidance of these possible accidents is a source of intense worry for all operators exposed in the handling of hypodermic needles, both for the extraction of blood and for the application of medicinal drugs, although presently these cases have diminished with the application of special hypodermic needles with anti-prick protection and special receiving boxes for disposal of the needle. For this reason the governments of the majority of Western countries are dictating standards for the use of materials comprising maximum security and avoiding these occupational accidents for the different health care operators exposed to these possible risks. Further information may be found, for example, in the study published by Dr Kennedy, of the Supplies Section of the U.K. Department of Health and Social Security.

Currently there are special containers for needle disposal and needles with protection means to protect from possible pricks, but none is practical nor do they allow the needle to be disposed of in a simple, easy, safe and economic way.

There is, therefore, the need to develop new systems allowing a blood sample having a good analytical quality, good metering, good aspiration without hemolysis, as well as good economy and hygienic handling for avoiding the risks existing in workers handling the blood sample to be obtained, where the quality of the analytical sample does not depend on the work skills of the operator making the extraction.

US 4.008.717 describes a small, portable, constant withdrawal device. It is connected to tubing, including a catheter, whose internal walls are coated with heparin. The catheter is inserted intravenously through a disposal needle into a subject such as a human being. The subject may then move about for a selected period when blood is being slowly withdrawn at a prescribed rate and collected in a container within a housing supporting the device. The collected blood may then be analyzed to permit the measurement of the integrated concentration of glucose, growth hormone or other material in blood. In addition, a portable microdiffusion chamber is incorporated between the indwelling catheter and the extra corporal tubing and is electrically connected through a sensor probe to an associated sensory responsive device. This permits continuous analysis of the withdrawn blood to determine the in vivo concentration of circulating concentrations of materials in the blood. WO 02/058556 describes a micro-sized device attached to the earlobe of a patient, for periodic, repeated analyses of blood or for injection of medicine. The device comprises a motor for moving a needle into the skin portion of the patient and withdrawal of a minute amount of blood for analysis. The result of the analysis controls the supply of a medical agent through the needle, whereupon the needle is retracted. The device is rotated over an angle before the next sampling, so that the new sampling takes place at a untouched skin area. US 5.046.509 describes an improved blood sampling device, which includes a coupling device connected to a needle for receiving blood withdrawn from a patient. The coupling device includes first and second ports in flow communication with the needle and is adapted to conduct blood to either the first or second port. The first port includes an adapter sleeve which is adapted to receive a vacutainer-type blood storage device for storing blood samples taken from the patient. A microporous hydrophobic plug is positioned within the coupling device intermediate the first port and the needle for venting air which is trapped within the coupling device during the initial stages of the sampling operation. The second port is connected to the coupling device intermediate the plug and the needle and is adapted to receive a syringe for temporarily storing a first portion of blood withdrawn from the patient thereby to ensure that blood subsequently delivered to the vacutainer contains gases having relative partial pressures and activities of ionic species substantially equal to those of the patient's in vivo conditions.

### Summary of the invention

It is an object of the invention to overcome these drawbacks. This purpose is achieved by a system for taking blood samples from a patient according to claim 6, characterized in that it comprises:
[i] a pump for extracting blood from a patient, where the pump is a peristaltic pump having a rotor, comprising at least one moving pumping surface and a fixed pumping surface, suitable for lodging therebetween a flexible tube, where the pump additionally comprises a head member suitable for the attachment of a sample holder tube support member, and
[ii] a blood sampling device according to claim 1 which, in turn, comprises [a] a needle holder having a tubular portion suitable for supporting a hypodermic needle having a connection base, the needle holder comprising a conduit with an inlet and an outlet, where said inlet comprises the tubular portion, [b] a sample holder tube support member and [c] a flexible tube having a first end in fluid communication with the needle holder and a second end in fluid communication with the support member.

Preferably, the sampling device additionally comprises a hypodermic needle with a connection base, the connection base being suitable for being mounted on the tubular portion.

Advantageously, the support comprises a cylindrical upper portion with a connector therein, where the cylindrical upper portion preferably has a plurality of openings. Advantageously, the support is made from a transparent material.

Preferably, the support member comprises at least two semi-cylindrical walls extending from the cylindrical upper portion, spaced apart by respective lateral openings, and very preferably it has four walls semi-cylindrical mutually separated by four lateral openings.

Advantageously, the connector comprises an end portion suitable for connection in fluid communication with the tubular portion. In this case, it is particularly advantageous that it comprises attachment means suitable for retaining the needle holder in the support member.

Preferably, the device comprises retaining means of the flexible tube.

Advantageously, the head member comprises an upper, horizontal U-shaped engagement portion and a lower portion having a smaller cross section than the upper portion.

Preferably, the head member comprises a sample holder tube presence sensor, a filling level sensor and/or a filling speed sensor.

Advantageously, the head member comprises spring retaining means of the support. Preferably, the pump comprises means for positioning the fixed surface relative to the moving surface suitable for moving the fixed surface between a spaced apart position and a pumping position, where the positioning means comprise reversible snap fitting means suitable for reversibly fixing the fixed surface relative to the pumping surface, and spring means urging said fixed surface to the spaced apart position.

Advantageously, the pump comprises electronic control means and a data viewing screen.

In general, the system preferably comprises a sampling device according to the invention, as described below.

In general, the system preferably comprises a pump for extracting blood according to the invention, as described below.

It is also described a pump for extracting blood from a patient characterized in that it is a peristaltic pump having a rotor, comprising at least one moving pumping surface and one fixed pumping surface, suitable for lodging therebetween a flexible tube, where the pump additionally comprises a head member suitable for the attachment of a sample holder tube support member.

In fact, the use of a peristaltic pump such as the indicated one for the extraction of blood from a patient offers a plurality of advantages:
1 - It allows always the extraction of an analytical blood sample under appropriate technical conditions, without hemolysis, without excessive vacuum, without overpressures, without incorrect metering, as well as the filling of various tubes with different volumes, etc., independently of the more or less correct behavior of the handler.
2 - It allows the extraction of any volume of blood, with the possibility of filling different types of container, with different volumes, even very small volumes (from 0.1 mm) or high volumes, for example, for blood banks, something which cannot be done with the vacuum system.
3 - The sample holder tube support member which will be attached to the head member may be designed in such a way as to be able to be used with any type of sample holder tube, of any format and fulfilling the requirements for any subsequent analysis or treatment.
4 - It may be used in any type of vein or artery, since it can use any type of needle of the great variety of standardized needles existing on the market, without the need of having to use the specific designs for vacuum or syringe-vacuum systems.
5 - It minimizes the hygienic risks (particularly if it is combined with the ejection system according to the invention), it is more practical and versatile (since it allows its use under any circumstances and in any vein), it is highly profitable and economic (particularly if it is combined with the use of sample holder tubes with the stopper marketed under the name of TAPVAL®.
6 - It is easily transportable, occupying little space. In this sense, it is advantageous for it to include a battery, such that the pump is an autonomous piece of equipment.
7 - It allows a plurality of functions to be automated, such as the reading of the analysis request, the whole extraction process the metering, the different tube formats, the labelling of the sample, etc.

Preferably the head member comprises an upper, horizontal U-shaped engagement portion and a lower portion having a smaller cross section than the upper portion. The semicircular portion of the U defines a longitudinal axis which, under the normal conditions of use of the pump, is preferably positioned vertically. After the sample holder tube support member with the corresponding sample holder tube has been mounted, the axis of the sample holder tube coincides with the longitudinal axis. The thus defined upper engagement portion allows for simple, effective assembly and disassembly of the sample holder tube support member, which moves along the arms of the U until it reaches the semicircular portion. The smaller cross section of the lower portion serves to hold the support member and to prevent it falling downwardly.

The head member advantageously comprises at least one of the following sensors:
1 - a sample holder tube presence sensor. The presence sensor is disposed preferably at the upper end, is advantageously optical and preferably detects the presence of the sample holder tube stopper (the sample holder tube is usually transparent, whereas the stopper is usually opaque). The axis of view advantageously passes through the support member, whereby there are two advantageous solutions for allowing the stopper to be detected: that the support member be made from transparent material or that it be provided with holes allowing the light from the sensor to pass through.
2 - a filling level sensor. This sensor is preferably an optical sensor also.
3 - a filling speed sensor. This sensor is advantageously likewise an optical sensor. It is preferably disposed under the drops of blood filling the tube and detects each of the falling drops. In this way, the number of drops falling per unit of time can be counted.

An advantageous alternative is for the filling level sensor and the filling speed sensor to share one same optical sensor. In fact, the optical sensor can discern between a falling drop and the full tube, since the full tube is much more opaque than a drop. Therefore, it is possible to distinguish between both phenomena with a single sensor.

Also the predetermined blood volume can be electronically metered, as can the extraction speed be programmed, the pump being automatically stopped when the programmed amount has been metered, giving a particular acoustic signal.

The head member preferably comprises retaining spring means for the support member. These spring means are advantageously protuberances projecting out from the arms of the U (and are orientated towards the inside of the U), in such a way that when the support member passes over them they retract and once the tube is in its definitive position, they extend again, (reversibly) retaining the support member in its position of use. In this way, the tube is prevented from being able to come out of its position of use undesirably, but it may be manually ejected, simply by overcoming the resilient force of the spring means.

During pumping, the extracted blood flows through the flexible tube disposed between the moving pumping surface and the fixed pumping surface. Therefore, it is necessary to change the flexible tube for each new patient. In this sense, it is particularly advantageous for the pump to be designed in such a way as to allow a quick, simple exchange of the used flexible tube with a new one. It must be borne in mind that the flexible tube will usually form part of a more complex assembly (see the sampling device described hereinafter). Thus, the pump preferably comprises positioning means for the fixed surface relative to the moving surface suitable for moving the fixed surface between a spaced-apart position, suitable for removing or positioning the flexible tube in the pump, and a pumping position. In fact, these positioning means allow the pump to be "opened" (when separating the moving surface from the fixed surface) and "closed" (when joining both pumping surfaces together. With the pump open, the flexible tube may be removed and replaced with a new one in an extremely simple way. The positioning means advantageously comprise reversible snap-fitting means for reversibly fixing the fixed surface relative to the pumping surface, and spring means urging said fixed surface to the spaced-apart position. The reversible snap fitting enormously simplifies the operation of "opening" and "closing" the pump, such that the operation of replacing the flexible tube may be done in a few seconds, without needing any special tool or specific knowledge or training.

The pump preferably comprises electronic control means and a data viewing screen.

As mentioned above, it is necessary to use a new flexible tube for each patient. In this sense, it is likewise an object of the invention to provide a sampling device characterized in that it comprises: [a] a needle holder having a tubular portion suitable for supporting a hypodermic needle having a connection base, said needle holder comprising a conduit with an inlet and an outlet, where said inlet comprises said tubular portion, [b] a sample holder tube support member and [c] a flexible tube having a first end in fluid communication with said needle holder and a second end in fluid communication with said support member. This whole device is replaced with a new one for each patient. It is therefore a consumable of a disposable kit, which may be used with the pump according to the invention. Advantageously, at the end of this support member, a security mechanism is connected to a self-sealing stopper therein allowing the air to pass for filling said device with simple venous pressure.

The sampling device preferably comprises, in addition, a hypodermic needle having a connection base, said connection base being suitable for being mounted on said tubular portion. In fact, although the needle could come separately, it is more convenient for the user if the needle comes already mounted in the needle holder.

The needle holder advantageously comprises an abutment surface and lever means suitable for separating the needle from the needle holder, the lever means comprising a lever having a first end suitable for being moved with a finger and a second end disposed between the connection base and the abutment surface. The needle holder advantageously comprises, furthermore, other improvements, such as for example:
- the needle holder comprises a flattened tab, suitable for being held between two fingers and extending from one of the sides of the conduit, where the flattened tab comprises the abutment surface and/or
- the lever means are integrally attached to said needle holder and are preferably attached to said flattened tab.

Alternatively, it is the needle connection base that comprises lever means suitable for separating the needle from the needle holder and the needle holder comprises an abutment surface, the lever means comprising a lever having a first end suitable for being moved with a finger and a second end disposed between the connection base and the abutment surface. In this case, the lever means are preferably integrally attached to the connection base.

A further possible alternative is for the sampling device according to the invention to comprise lever means suitable for separating the needle from the needle holder which form an independent part with attachment means suitable for attaching the independent part to the tubular portion and between the connection base and the abutment surface, where the needle holder comprises an abutment surface and where the lever means comprise a lever having a first end suitable for being moved with a finger and a second end disposed between the connection base and the abutment surface. These attachment means are preferably a ring or a horseshoe-shaped protuberance.

The lever is preferably rotatably attached to the needle holder, to the connection base or to the ring at an intermediate point, where the lever forms an angle of more than 45° with the needle axis, advantageously more than 70°.

The tubular portion preferably defines a Luer connection according to ISO 594.1986, DIN 1707:1996 or DIN 20594-1:1993.

The support member advantageously comprises a cylindrical upper portion having a connector in the interior thereof. The cylindrical upper portion is thus particularly suitable for engaging the upper, horizontal U-shaped engagement portion of the head member of the pump according to the invention. In turn, the inner connector allows for connection to the sample holder tube. As already commented hereinbefore, a particularly advantageous solution is for the cylindrical upper portion to have a plurality of openings, through which the light of a sample holder tube presence sensor can propagate. Likewise, an alternative is for the support member to be made from a transparent material. When indicating 'transparent", it must be understood to be transparent at least for the frequency of the light emitted by the corresponding sensor.

The support member preferably comprises at least two semi-cylindrical walls extending from the cylindrical upper portion, spaced apart by respective lateral openings, and very preferably it has four walls semi-cylindrical separated by four lateral openings. In fact, the presence of the lateral openings allows the support member to be positioned in the head member in a pre-established position, such as will be seen in the embodiments described hereinafter.

Advantageously, the connector disposed in the interior of the cylindrical upper portion of the support member advantageously comprises an end portion suitable for connection in fluid communication with the tubular portion. In fact, once the sampling device according to the invention has been used, it is removed from the pump and the hypodermic needle is disposed of. Nevertheless, the tube still contains blood. It is therefore necessary to contemplate a way of preventing this blood from representing a risk. The proposed solution allows the tubular portion of the needle holder to be connected to the end portion of the connector, thanks to which a closed circuit is formed in such a way that the blood preset in the flexible tube no longer flows out of it. In this way the used sampling device may be deposited in any conventional container, without any risk of spilling blood. The sampling device preferably comprises attachment means suitable for retaining the needle holder in the support member. In fact, a simple interference fit between the tubular portion and the end portion may be insufficient, such that the presence of attachment means reinforcing this joint may be of interest. In this sense, it is of particular interest that these attachment means comprise a groove disposed in the lever means. This groove may form an interference fit with the semi-cylindrical walls, as will be appreciated more clearly when explaining hereinafter embodiments of the invention.

The sampling device advantageously comprises means for retaining the flexible tube such that the flexible tube does not move relative to the pump when the pump is pumping the blood. For example, the flexible tube may comprise a ring disposed therearound at a pre-established position and fixed thereto and the pump may have a guide through which the naked tube, but not the ring, can pass. Thus, it may also be achieved that these flexible tube retaining means also act as means for the correct positioning of the flexible tube, thereby preventing the position from being reversed. Thus a functional security means is included in the assembly preventing the pump from aspirating in a direction opposed to the right one.

It is also described a hypodermic needle having a connection base characterized in that it comprises an ejection device integrally attached to the connection base, where the ejection device comprises lever means provided with a lever having a first end suitable for being moved with a finger and a second end in the proximity of the connection base. The lever means preferably include at least one of the other preferred solutions described in the present specification.

It is also described a syringe comprising a hollow cylindrical body having a front wall, closed except for an outlet orifice from which a tubular portion extends, and a plunger suitable for traversing the interior of the hollow cylindrical body, characterized in that it comprises an ejection device integrally attached to the front wall, where the ejection device comprises lever means provided with a lever having a first end suitable for being moved with a finger and a second end in the proximity of the tubular portion. The lever means preferably include at least one of the other preferred solutions described in the present specification.

It is also described a needle holder comprising a conduit having an inlet and an outlet, where the inlet comprises a tubular portion, characterized in that it comprises an ejection device integrally attached to the needle holder, where the ejection device comprises lever means provided with a lever having a first end suitable for being moved with a finger and a second end in the proximity of the tubular portion. The lever means preferably include at least one of the other preferred solutions described in the present specification.

In general, the fact of having said lever means allows ejecting the hypodermic needle in a very simple way, without the risk of being pricked and without the need for special containers and/or protectors. The user holds the syringe or needle holder (or, in general, any holder device having a tubular portion on which it must be possible to mount the connection base of the syringe) in the hand in the same position as in which the needle had been withdrawn from the patient's body such that the needle tip remains remote from the hand. With one finger of the same hand, the operator moves the first end of the lever and the second end exerts the force required for separating the connection base from the abutment, thereby ejecting the needle from the carrying device. Prior to performing this ejection movement with his or her finger, the user directs the tip of the needle towards a container for sharp-edged objects (which may be fully conventional in accordance with the current regulations) and on performing the ejection movement, the needle is projected towards the container. At all times the user's hand is behind the needle tip and the user's other hand should not participate in any way in the ejection procedure. Thus, the risk of being pricked is totally eliminated; the container may be a completely conventional container in keeping with current regulations, i.e. not having any special features for the ejection of the needle.

Generally speaking, the hypodermic needle comprises always a fine tube with a sharp tip at one end (which is the one inserted into the patient's body) and with the other end integrated in a connection base. The connection base allows the needle to be connected to any carrying device, such that there is established a fluid connection between the inside of the needle and the inside of the tubular portion of the carrying device. In general, any type of connection may be established between the connection base and the tubular portion, with the sole limitation that it must be a reversible connection when applying a force tending to separate the connection base from the tubular portion, since this will be the force which is exerted by the lever. The tubular portion preferably defines a Luer connection according to ISO 594.1986, DIN 1707:1996 or DIN 20594-1:1993. In fact, the so-called Luer connections are widely used, whereby the ejection device according to the invention may advantageously be applied to hypodermic needles having a Luer type connection.

There are several solutions, all advantageous, for integrating the lever means in the ejection device. Thus, they may be integrally attached to the carrying device or they may be integrally attached to the connection base. In these cases, and since the carrying device and the connection base are usually made from plastics materials, it is advantageous for the lever means to form an integral unit with the carrying device or with the connection base, directly obtainable from the plastics injection mould. The lever means may also advantageously be an independent part having attachment means suitable for attaching the independent part to the tubular portion between the connection base and the abutment surface. These attachment means are preferably a ring or a horseshoe-shaped protuberance.

The carrying device may be any one; the sole limitation being that it must have a tubular portion on which to be able to mount the connection base. Depending on the type of carrying device in question, various preferred embodiments of the invention may be obtained:
1 - The carrying device may be a syringe, having a hollow cylindrical body with a front wall, closed except for an outlet orifice from which the tubular portion extends, and a plunger suitable for traversing the interior of the hollow cylindrical body, where the front wall comprises an abutment surface. The second end of the lever is disposed between the connection base and the abutment surface.
   In this case, it is particularly advantageous for the lever means to be integrally attached to the syringe, and they are preferably attached to the front wall. Nevertheless, the other alternatives (lever means attached to the connection base or lever means as an independent part) are also viable.
2 - The carrying device may be a needle holder, comprising a conduit having an inlet and an outlet, where the inlet comprises the tubular portion and an abutment surface. The second end of the lever is disposed between the connection base and the abutment surface. Normally, the needle holder outlet is, in turn, connected (in fluid communication) to another member, for example a tube, allowing the blood to be transferred to the desired place. To facilitate the handling of the needle holder, it advantageously comprises a flattened tab to be held between two fingers and extending from one of the two sides of the conduit, where the flattened tab comprises the abutment surface. It is thus possible to design a needle holder allowing the needle to be positioned very tangentially to the users skin and which, in spite of this, may be easily handled thanks to the tab. It is also particularly advantageous in this case for the lever means to be integrally attached to the needle holder. They are preferably attached to the flattened tab. The other alternatives (lever means attached to the connection base or lever means as an independent part) are also viable in this case.

Generally speaking, it is advantageous for the lever to be rotatably attached to the carrying device, to the syringe, to the needle holder, to the connection base or to the ring at an intermediate point, where the lever forms an angle of more than 45°with the axis of the needle, preferably more than 70°. Thus, the first end of the lever protrudes noticeably away from the carrying device and the connection base, such that it is easily accessible for the finger.

In general, the operative mechanism will be as follows:
- if the lever means are on the connection base of the hypodermic needle, when the first end of the lever is moved forwards, the second end moves backwards until it contacts the abutment surface of the syringe or needle holder. From this time onwards, the forward movement of the first end will cause the needle to be separated from the syringe or needle holder.
- if the lever means are on the front wall of the syringe or on the needle holder, when the first end of the lever is moved backwards, the second end moves forwards until it contacts the connection base of the needle. From this time onwards, the backward movement of the first end will cause the needle to be separated from the syringe or needle holder.
- if the lever means are an independent part disposed on the tubular portion of the syringe or the needle holder and between the connection base and the front wall of the syringe or the rest of the body of the needle holder, when the first end of the lever is moved backwards, the second end moves forwards until the second end contacts the connection base of the needle and an intermediate point of the lever contacts the abutment surface (for this the lever means may have to move along thee tubular portion). From this time onwards, the backward movement of the first end will cause the needle to be separated from the syringe or needle holder.

### Brief description of the drawings

Further advantages and features of the invention will become apparent from the following description, in which, without any limiting character, preferred embodiments of the invention are disclosed, with reference to the accompanying drawings in which:
Figure 1 is a front perspective view of a first ejection device.
Figure 2 is a rear perspective view of the ejection device of Figure 1.
Figure 3 is a side elevation view of the ejection device of Figure 1.
Figure 4 is a front perspective view of the ejection device of Figure 1 mounted on a syringe.
Figure 5 is a side elevation view of a second ejection device.
Figure 6 is a front perspective view of the ejection device of Figure 5.
Figure 7 is a side elevation view of a third ejection device.
Figure 8 is a side elevation view of a fourth ejection device.
Figure 9 is a perspective view of a sampling device according to the invention.
Figure 10 is a perspective view of the sampling device of Figure 9 mounted on a pump for the extraction of blood according to the invention with the positioning means in an open position.
Figure 11 is the same view as Figure 10, but with the positioning means in a closed position.
Figure 12 is a side elevation view of the pump of Figure 10, with the positioning means in an open position.
Figure 13 is a side elevation view of the pump of Figure 10, with the positioning means in a closed position.
Figure 14 is a top perspective view of the pump of Figure 10, with the positioning means in an open position.
Figure 15 is a lower front perspective view of a head member according to the invention.
Figure 16 is a front elevation view of a support member according to the invention.
Figure 17 is a lower front perspective view of the support member of Figure 16 mounted in the head member of Figure 15.
Figures 18 and 19 are views of a sampling device having the needle holder mounted in the connector of the sample holder support member.
Figure 20 is a view equivalent to that of Figure 17 where the support has a security mechanism.

### Detailed description of embodiments of the invention

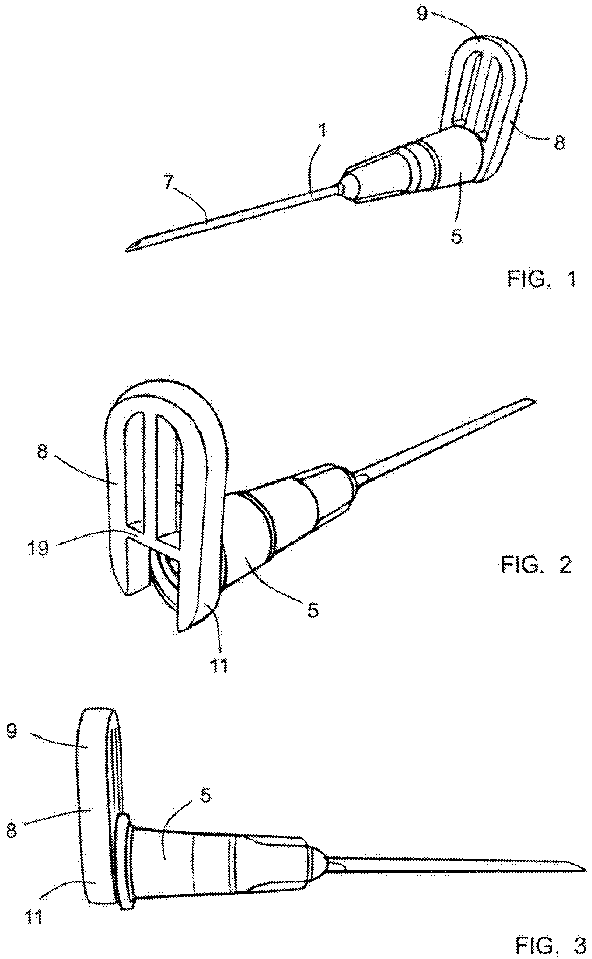

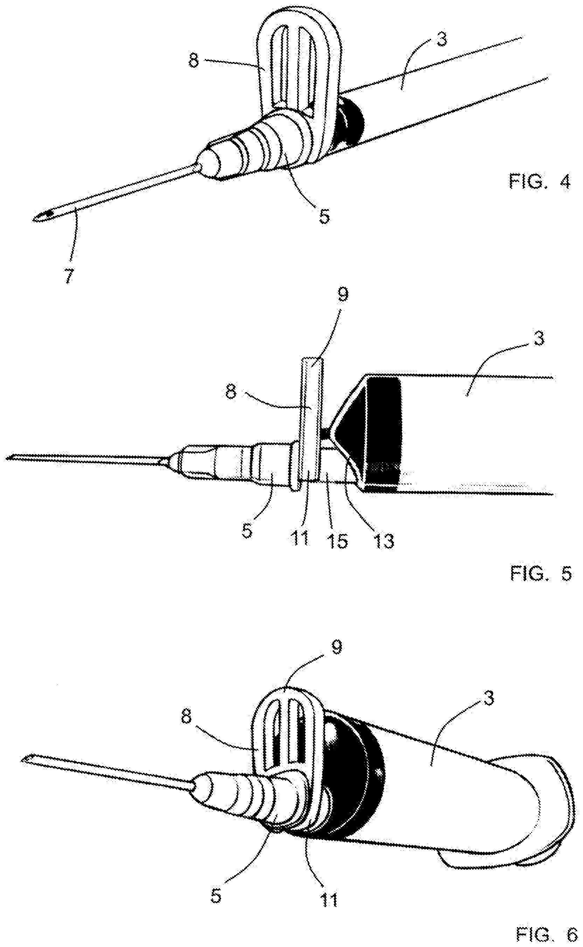

In Figures 1 to 4, there is shown a first embodiment of an ejection device for a hypodermic needle 1 suitable for ejecting the hypodermic needle 1 from a carrying device 3 for the hypodermic needle 1 according to the invention. The hypodermic needle 1 has a connection base 5 and a tube 7 having a sharp-pointed end. In this first embodiment, the ejection device is integrally attached to the connection base 5. The ejection device is provided with lever means having a lever 8 which is attached to the connection base 5. The lever 8 has a first end 9 remote from the connection base 5 and a second end 111 which is disposed between the connection base 5 and the front wall 13 of the carrying device 3 which, in this particular case, is a conventional syringe. The syringe has a hollow cylindrical body with a front wall 13 having an outlet orifice from which there extends a tubular portion 15 (not visible in Figures 1 to 4, but visible, for example, in Figure 5) on which the connection base 5 of the needle 1 is mounted. The front wall 13 acts as an abutment surface 17. In Figure 2 there are to be seen in greater detail the lever means: the lever 8 has the general form of an inverted U having a transverse partition 9 which connects the arms of the U at an intermediate point. The lever means are attached to the connection base 5 through this partition 19, such that they may rock about the joint area between the connection base 5 and the partition 19.

The user of the syringe, to eject the hypodermic needle 1 from the syringe, has only to move the first end 9 of the lever 8 in a forward direction (namely, push it with a finger to separate it from the syringe) in this way the second end 11 moves towards the front wall 13 of the syringe and urges the connection base 5 in an outward direction. Thus, with a movement of one finger of the same hand that is holding the syringe the needle 1 may be easily ejected.

In Figures 5 and 6, there is shown a second embodiment of an ejection device for a hypodermic needle. In this case, the transverse partition 19 is attached to the front wall 13 of the syringe. Therefore, in this case, to eject the needle 1, the user must push the first end 9 in a backward direction. Thus, the second end 11 will move in a forward direction and will eject the needle 1. It is of particular interest when using syringes with an eccentric cone.

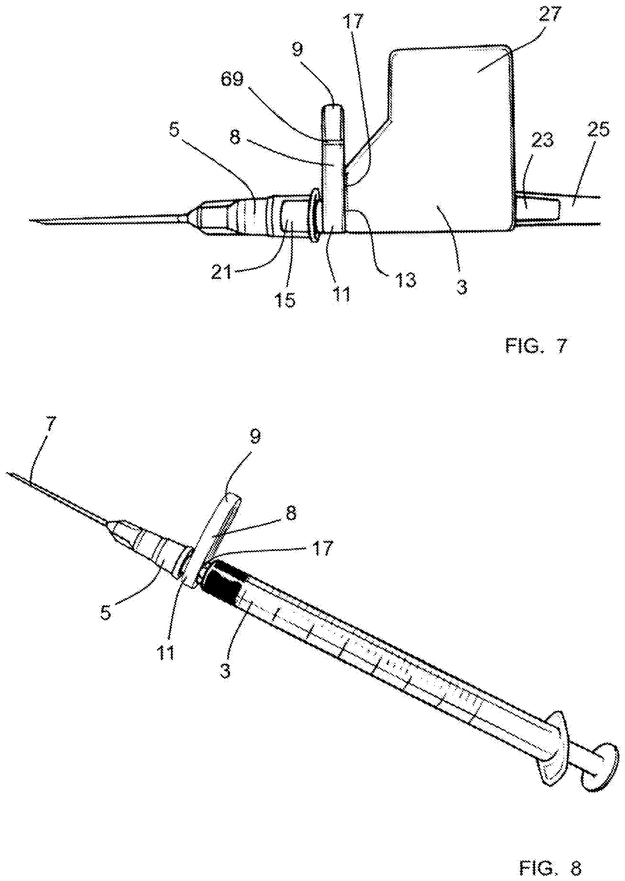

Figure 7 shows a third embodiment of an ejection device for a hypodermic needle 1. In this example, the needle carrying device 3 for the hypodermic needle 1 is not a syringe, but is a needle holder. The needle holder has a conduit with an inlet 21 and an outlet 23. The inlet 21 defines a tubular portion 15 on which the needle 1 is mounted. The outlet 23 is connected to a flexible tube 25 allowing the extracted blood to be taken to wherever corresponds. The needle holder comprises, additionally, a flattened tab 27 extending from one of the sides of the conduit, serving to be held with the fingers. The needle holder is also provided with an ejection device consisting of lever means such as those already described. In this case, the transverse partition 19 is attached to the front wall 13 of the needle holder, which performs the function of abutment surface 17. As may be seen in Figure 7, the needle holder is clearly asymmetrical, in the sense that the user may hold it with two fingers by way of the tab 27 and can position the needle 1 very tangentially relative to the patient's skin.

Figure 8 shows another embodiment of an ejection device according to the invention. In this case, the ejection device is an independent part having a ring which is disposed on the tubular portion 15 of the syringe between the connection base 5 of the needle 1 and the front wall 13 of the syringe defining the abutment surface 17.

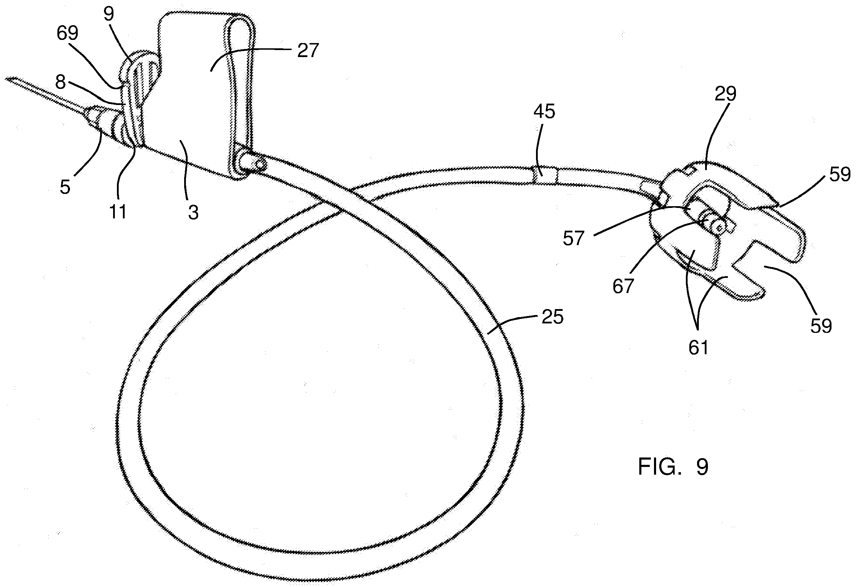

In Figure 9 there is shown a sampling device according to the invention. The sampling device comprises a flexible tube 25 having connected thereto at a first end a needle holder such as the one shown in Figure 7 and at the opposite end a support member 29 for sample holder tubes. In the embodiment of Figure 9, the sampling device also includes a hypodermic needle 1 and an ejection device having lever means such as those described hereinbefore.

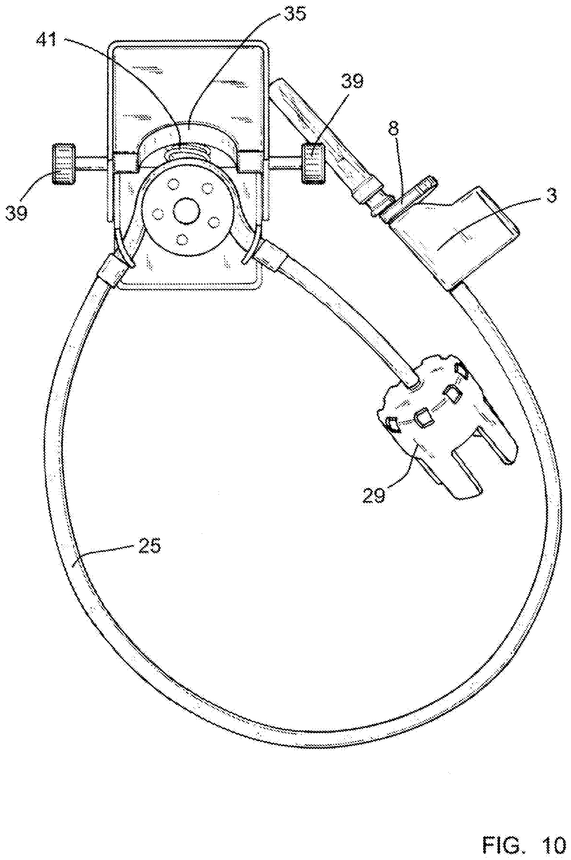

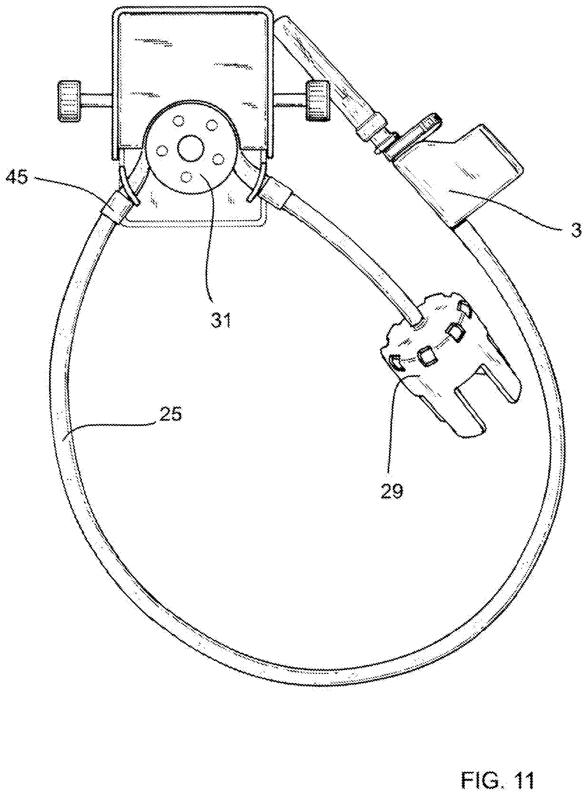

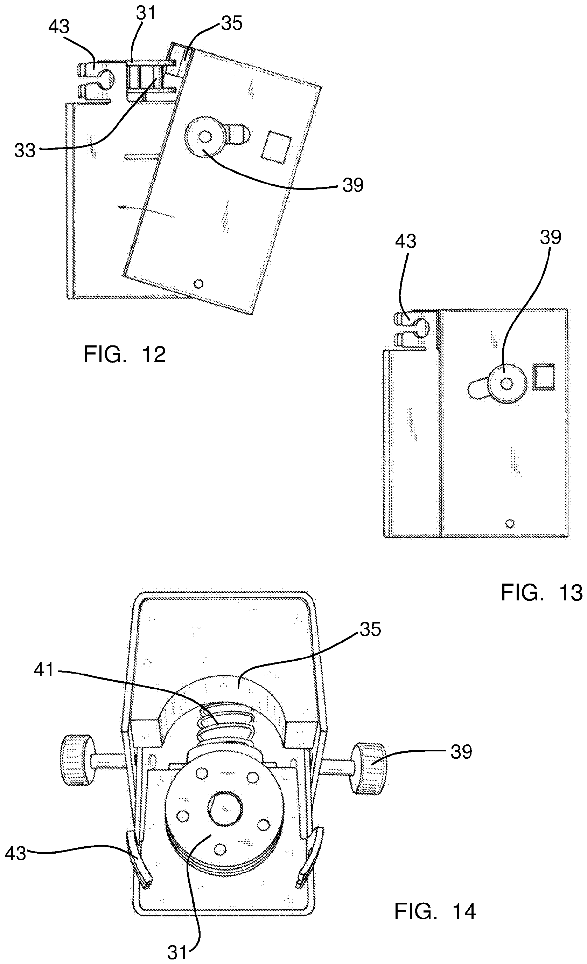

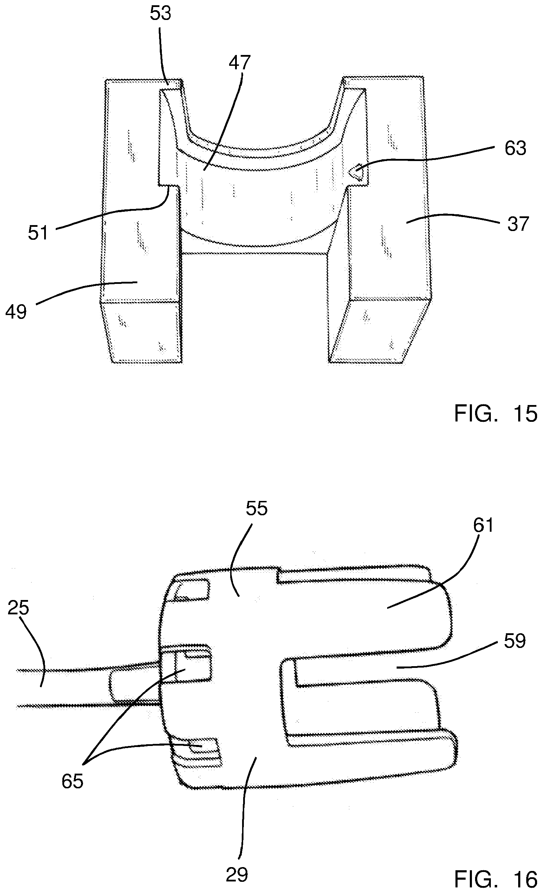

In Figures 10 to 14, there is shown a pump for extracting blood from a patient according to the invention. The pump comprises a peristaltic pump having a rotor 31 provided with a moving pumping surface 33 (with the lobes characteristic of a peristaltic pump) and a fixed pumping surface 35. The pump also has a head member 37 (see Figure 15) suitable for attachment of a support member 29 of a sample holder tube. In fact, the pump has a chassis in which both the peristaltic pump and the head member 37 are attached. Nevertheless, in the figures the peristaltic pump and the head member 37 are shown separated from the pump chassis. The pump also has positioning means for the fixed surface 35 relative to the moving surface 33 suitable for moving the fixed surface 35 between a spaced apart position (see Figure 10, the positioning means are in the "open" position), in which the fixed surface 35 is spaced apart from the moving surface 33 and a pumping position (see Figure 11, the positioning means are in the "closed" position), in which the fixed surface 35 is in the proximity of the moving surface 33, i.e. it is at the appropriate distance to be able to perform the pumping function. When the positioning means are in the open position, there is positioned between both pumping surfaces 33 and 35 the flexible tube 25 in such a way that when the positioning means are in a closed position the flexible tube 25 is compressed between both and the function of pumping or of a seal (preventing the blood from flowing out under the venous pressure itself) may be performed.

The pump has two buttons 39 for operating the reversible snap fit means. When the buttons 39 are pressed, a metal strip is elastically deformed and thus there is released a step portion disposed on the fixed part of the pump which was lodged in a housing provided in the moving part of the pump. Spring means 41 (in the form of a spiral spring) urge the moving part of the pump (comprising the moving surface 33) towards the open position. To close the pump (for example, after positioning a new flexible tube 25), it is sufficient to push the moving part towards the fixed part until the step portion relodges itself in the housing.

The pump has guide means 43 for the flexible tube 25 which also serve to hold the flexible tube 25 in a predetermined position thanks to the retaining means 45 disposed in the flexible tube 25 (in the form of a ring disposed on the flexible tube 25 and whose outer diameter is greater than the gap in the guide means 43.

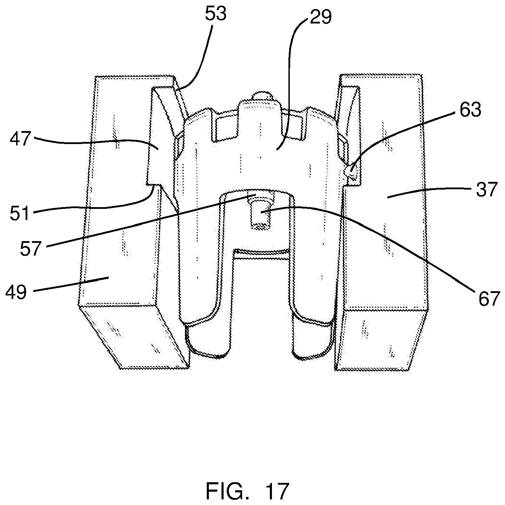

Figure 15 shows a head member 37 suitable for the attachment of a support member 29 for a sample holder tube. The head member 37 comprises an upper engagement portion 47, in the form of a horizontal U and a lower portion 49 having a cross section smaller than that of the upper portion. Thus there is formed a step portion 51 which will prevent the support member 29 from moving in a downward direction (see Figure 17). The upper portion 47 is also provided with an upper step portion 53, such that the support member 29 cannot move in an upward direction either.

Figure 16 shows a support member 29 for a sampling device according to the invention. It comprises a cylindrical upper portion 55 of the same size as the upper portion 47 of the engagement head member 37, such that it is suitable for being lodged in the interior of the upper engagement portion 47 by inserting it along the horizontal arms of the U (see Figure 17). The support member 29 has a connector 57 in the interior thereof, disposed coaxially with the cylindrical upper portion 55. Following the cylindrical upper portion 55, the cylindrical surface has four lateral openings 59, such that the cylindrical surface extends as four semi-cylindrical walls 61. Thus, when lining up the lateral openings 59, the support member 29 can be inserted in the head member 37 in a predefined position.

In Figure 17, there is shown the support member 29 lodged in the head member 37. The head member 37 has spring retaining means 63 for the support member 29 disposed in the upper engagement portion 47 and projecting out relative to the centre of the semi-circumference of the U in the direction of the arms of the U. Thus, when the cylindrical upper portion 55 of the support member is pushed towards the bottom of the U, the spring retaining means 63 are forced backwards and once the support member 29 is in its final position of use, then the spring retaining means 63 project once again towards the interior of the U, such that the support member 29 cannot come out of its final position without overcoming the force exerted by these spring retaining means 63.

The support member 29 also has a plurality of openings 65 in the cylindrical upper portion 55. Thus, when the support member 29 is lodged in the head member 37, a presence sensor present in the head member 37 is situated facing two of these openings 65, such that a beam of light can pass through the support member 29. In the case where the support member 29 has a sample holder tube mounted thereon, the stopper of the sample holder tube is disposed between the orifices 65, such that the beam of light emitted by the sensor cannot reach the corresponding receiver, thereby detecting the presence of the sample holder tube and preventing the pump from being started if there is no sample holder tube to collect the blood.

The head member 37 may include a filling level sensor which is at the height of the lateral openings 59. In a way similar to the previous case, a beam of light passes through the sample holder tube, such that it may be detected when the blood level reaches the height of the corresponding sensor.

The head member 37 may likewise include a filling speed sensor, which is also at the height of the lateral openings 59 and positioned just below the connector 57. Thus, it is possible to detect the drops of blood falling to fill the sample holder tube. These drops can be counted, which allows knowledge of the volume and filling speed of the sample holder tube to be known in real time. Thus, the speed of rotation of the peristaltic pump may be adjusted, for example to avoid problems of generation of a vacuum and possible hemolysis of the blood sample. The filling level sensor and the filling speed sensor may physically be one same sensor having both functions, since it is possible to distinguish between a falling drop and a tube which is full thanks to the fact that the amount of light absorbed is different in one case and the other.

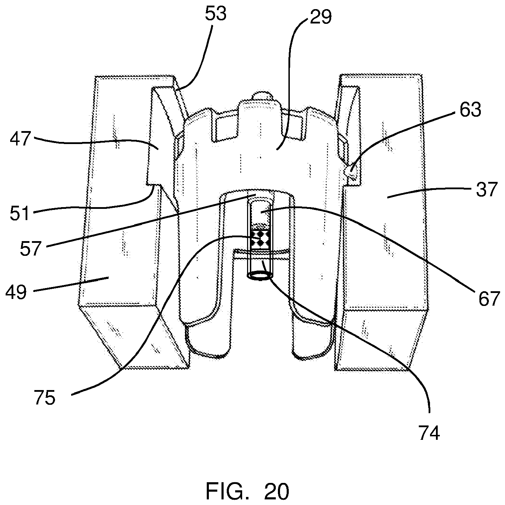

In Figure 20, there is shown the case in which the support member 29 has, at the end, a security mechanism 74, shown in Figure 20 as it was made from a transparent material, in order to be able to see that therein it has a self-sealing stopper (75). The self-sealing stopper (75) allows the air to pass for filling the device by simple venous pressure.

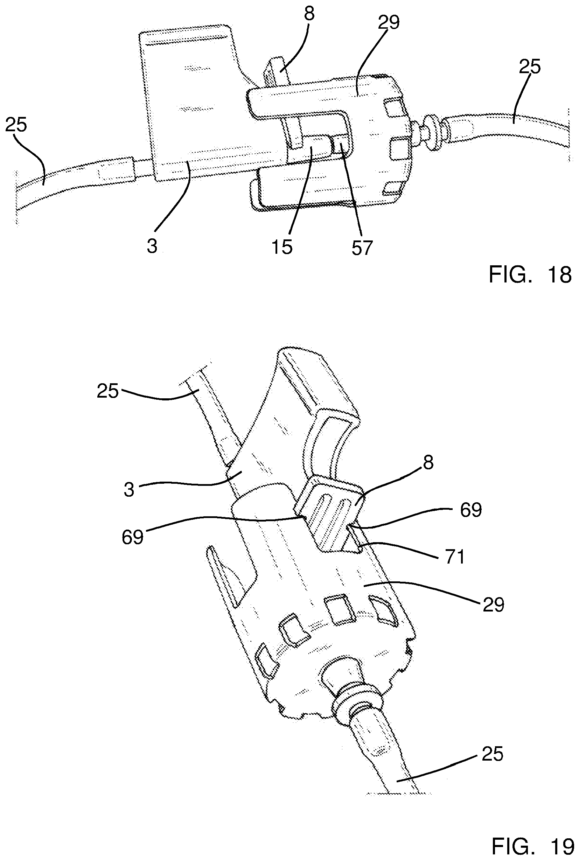

In Figures 18 and 19 there is shown a sampling device with the needle holder mounted on the connector 57 of the support member 29. To this end, the connector 57 of the support member 29 has an end portion 67 which is suitable for engaging in the interior of the tubular portion 15. Thus, once the hypodermic needle 1 has been ejected, it is possible to mount the needle holder on the connector 57, such that the flexible tube 25 is closed on itself, whereby possible spilling of the blood remaining in the interior thereof is avoided. Furthermore, the lever means comprise attachment means, in the form of a groove 69, for guiding, which frictionally slides on the side edge 71 of the semi-cylindrical walls 61. Thus, it is ensured that the needle holder will not easily become separated from the connector 57.

## Claims

1. Blood sampling device that comprises: [a] a needle holder having a tubular portion (15) suitable for supporting a hypodermic needle (1) having a connection base (5), said needle holder comprising a conduit with an inlet and an outlet, where said inlet comprises said tubular portion (15), [b] a sample holder tube support member (29) and [c] a flexible tube (25) having a first end in fluid communication with said needle holder and a second end in fluid communication with said support member (29),
where the support member (29) comprises a cylindrical upper portion (55) having a connector (57) in the interior thereof, and said connector (57) comprises an end portion (67) suitable for connection in fluid communication with said tubular portion (15),
**characterized in that**
[d] said needle holder comprises an abutment surface (17) and lever means suitable for separating the needle (1) from the needle holder, said lever means comprising a lever (8) having a first end (9) suitable for being moved with a finger and a second end (11) disposed between said connection base (5) and said abutment surface (17), where said needle holder comprises a flattened tab (27) to be held between two fingers and extending from one of the two sides of the conduit, where said flattened tab (27) comprises said abutment surface (17), and where said lever means are integrally attached to said needle holder,
[e] said connector (57) of the support member (29) has an end portion (67) which is suitable for engaging in the interior of the tubular portion (15),
[f] said support member (29) comprises at least two semi-cylindrical walls (61) extending from the cylindrical upper portion, mutually separated by lateral openings (59),
[g] said lever (8) comprises a groove (69), where said groove (69) is able to frictionally slide on the side edge (71) of the semi-cylindrical walls (61).

2. Blood sampling device according to claim 1, **characterized in that** it additionally comprises a hypodermic needle (1) having a connection base (5), said connection base (5) being suitable for being mounted on said tubular portion (15).

3. Blood sampling device according to claim 1, **characterized in that** said cylindrical upper portion (55) has a plurality of openings.

4. Blood sampling device according to any one of claims 1 to 3, **characterized in that** the support member (29) comprises four semi-cylindrical walls (61) mutually separated by four lateral openings (59).

5. Blood sampling device according to any one of claims 1 to 4, **characterized in that** at the end of said support member (29) a security mechanism (74) is connected to a self-sealing stopper (75) therein suitable for allowing the air to pass for filling said device with simple venous pressure.

6. A system for taking blood samples from a patient, **characterized in that** it comprises:
[i] a pump for extracting blood from a patient, where said pump is a peristaltic pump having a rotor (31) comprising at least one moving pumping surface (33) and a fixed pumping surface (35), suitable for lodging therebetween a flexible tube (25), where the pump additionally comprises a head member (37) suitable for the attachment of a sample holder tube support member (29), where the head member (37) comprises an upper engagement portion (47), in the form of a horizontal U and a lower portion (49) having a cross section smaller than that of the upper portion (47), and
[ii] a blood sampling device according to any one of claims 1 to 5, where said cylindrical upper portion (55) is of the same size as said upper engagement portion (47) of the head member (37), such that it is suitable for being lodged in the interior of the upper engagement portion (47) by inserting it along the horizontal U.

7. System according to claim 6, **characterized in that** the head member (37) comprises a sample holder tube presence sensor, a filling level sensor and/or a filling speed sensor.

8. System according to one of claims 6 or 7, **characterized in that** the head member comprises spring retaining means (63) for the support member (29).

9. System according to any one of claims 6 to 8, **characterized in that** it comprises means for positioning the fixed surface (35) relative to the moving surface (33) suitable for moving the fixed surface (35) between a spaced apart position and a pumping position, where the positioning means comprise reversible snap fitting means suitable for reversibly fixing the fixed surface (35) relative to the pumping surface, and spring means urging said fixed surface (35) to the spaced apart position.

10. System according to any one of claims 6 to 9, **characterized in that** it comprises electronic control means and a data viewing screen.

11. System according to any one of claims 6 to 10, **characterized in that** at the end of said support member (29) a security mechanism (74) is connected to a self-sealing stopper (75) therein suitable for allowing the air to pass for filling said device with simple venous pressure.

## Patentansprüche

1. Blutprobenvorrichtung, die Folgendes umfasst: [a] einen Nadelhalter mit einem röhrenförmigen Abschnitt (15), der zum Stützen einer Injektionsnadel (1) mit einer Verbindungsbasis (5) geeignet ist, wobei der Nadelhalter eine Leitung mit einem Einlass und einem Auslass umfasst, wobei der Einlass den röhrenförmigen Abschnitt (15) umfasst, [b] ein Probenhalterrohr-Stützelement (29) und [c] einen Schlauch (25) mit einem ersten Ende in Fluidverbindung mit dem Nadelhalter und einem zweiten Ende in Fluidverbindung mit dem Stützelement (29), wobei das Stützelement (29) einen zylindrischen oberen Abschnitt (55) mit einem Verbinder (57) in seinem Inneren umfasst und der Verbinder (57) einen Endabschnitt (67) umfasst, der zur Verbindung in Fluidverbindung mit dem rohrförmigen Abschnitt (15) geeignet ist,
**dadurch gekennzeichnet, dass**
[d] der Nadelhalter eine Widerlagerfläche (17) und eine Hebeleinrichtung umfasst, die zum Trennen der Nadel (1) vom Nadelhalter geeignet sind, wobei die Hebeleinrichtung einen Hebel (8) mit einem ersten Ende (9) umfasst, das zum Bewegen mit einem Finger geeignet ist, und ein zweites Ende (11), das zwischen der Verbindungsbasis (5) und der Widerlagerfläche (17) angeordnet ist,
wobei der Nadelhalter einen abgeflachten Vorsprung (27) umfasst, der zwischen zwei Fingern gehalten werden soll und sich von einer der zwei Seiten der Leitung erstreckt, wobei der abgeflachte Vorsprung (27) die Widerlagerfläche (17) umfasst und wobei die Hebeleinrichtung einteilig an dem Nadelhalter angebracht ist,
[e] der Verbinder (57) des Stützelements (29) einen Endabschnitt (67) aufweist, der zum Eingreifen in das Innere des röhrenförmigen Abschnitts (15) geeignet ist,
[f] das Stützelement (29) mindestens zwei halbzylindrische Wände (61) umfasst, die sich von dem zylindrischen oberen Abschnitt erstrecken und durch laterale Öffnungen (59) voneinander getrennt sind,
[g] der Hebel (8) eine Nut (69) umfasst, wobei die Nut (69) an dem Seitenrand (71) der halbzylindrischen Wände (61) formschlüssig gleiten kann.

2. Blutprobenvorrichtung nach Anspruch 1, **dadurch gekennzeichnet, dass** diese zusätzlich eine Injektionsnadel (1) mit einer Verbindungsbasis (5) umfasst, wobei die Verbindungsbasis (5) zur Installation an dem röhrenförmigen Abschnitt (15) geeignet ist.

3. Blutprobenvorrichtung nach Anspruch 1, **dadurch gekennzeichnet, dass** der zylindrische obere Abschnitt (55) eine Vielzahl von Öffnungen aufweist.

4. Blutprobenvorrichtung nach einem der Ansprüche 1 bis 3, **dadurch gekennzeichnet, dass** das Stützelement (29) vier halbzylindrische Wände (61) umfasst, die durch vier laterale Öffnungen (59) voneinander getrennt sind.

5. Blutprobenvorrichtung nach einem der Ansprüche 1 bis 4, **dadurch gekennzeichnet, dass** am Ende des Stützelements (29) ein Sicherheitsmechanismus (74) mit einem selbstdichtenden Stopfen (75) darin verbunden ist, der geeignet ist, die Luft durchzulassen, um die Vorrichtung mit einfachem Venendruck zu füllen.

6. System zum Nehmen von Blutproben von einem Patienten, **dadurch gekennzeichnet, dass** es umfasst:
[i] eine Pumpe zum Extrahieren von Blut von einem Patienten, wobei die Pumpe eine peristaltische Pumpe mit einem Rotor (31) ist, der mindestens eine sich bewegende Pumpfläche (33) und eine feste Pumpfläche (35) umfasst, die zum Unterbringen eines flexiblen Rohrs (25) dazwischen geeignet ist, wobei die Pumpe zusätzlich ein Kopfelement (37) umfasst, das zur Befestigung eines Probenhalterrohr-Stützelements (29) geeignet ist, wobei das Kopfelement (37) einen oberen Eingriffsabschnitt (47) in der Form eines horizontalen U und einen unteren Abschnitt (49) mit einem Querschnitt, der kleiner als der des oberen Abschnitts (47) ist, umfasst, und
[ii] eine Blutprobenvorrichtung nach einem der Ansprüche 1 bis 5, wobei der zylindrische obere Abschnitt (55) die gleiche Größe wie der obere Eingriffsabschnitt (47) des Kopfelements (37) aufweist, so dass dieser zum Unterbringen im Inneren des oberen Eingriffsabschnitts (47) durch Einsetzen entlang des horizontalen U geeignet ist.

7. System nach Anspruch 6, **dadurch gekennzeichnet, dass** das Kopfelement (37) einen Probenhalterrohr-Anwesenheitssensor, einen Füllstandsensor und/oder einen Füllgeschwindigkeitssensor umfasst.

8. System nach einem der Ansprüche 6 oder 7, **dadurch gekennzeichnet, dass** das Kopfelement Federrückhalteeinrichtungen (63) für das Stützelement (29) umfasst.

9. System nach einem der Ansprüche 6 bis 8, **dadurch gekennzeichnet, dass** es Mittel zum Positionieren der festen Fläche (35) in Bezug auf die sich bewegende Fläche (33) umfasst, die zum Bewegen der festen Fläche (35) zwischen einer beabstandeten Position und einer Pumpposition geeignet ist, wobei die Positionierungseinrichtungen reversible Schnappverbindungseinrichtungen umfassen, die zum reversiblen Befestigen der festen Fläche (35) relativ zur Pumpfläche geeignet sind, und Federeinrichtungen, welche die feste Fläche (35) in die beabstandete Position drücken.

10. System nach einem der Ansprüche 6 bis 9, **dadurch gekennzeichnet, dass** es elektronische Steuereinrichtungen und einen Datenansichtsbildschirm umfasst.

11. System nach einem der Ansprüche 6 bis 10, **dadurch gekennzeichnet, dass** am Ende des Stützelements (29) ein Sicherheitsmechanismus (74) mit einem selbstdichtenden Stopfen (75) darin verbunden ist, der geeignet ist, die Luft durchzulassen, um die Vorrichtung mit einfachem Venendruck zu füllen.

## Revendications

1. Dispositif de prélèvement sanguin qui comprend : [a] un porte-aiguille ayant une partie tubulaire (15) apte à supporter une aiguille hypodermique (1) ayant une base de raccordement (5), ledit porte-aiguille comprenant un conduit avec une entrée et une sortie, où ladite entrée comprend ladite partie tubulaire (15), [b] un élément de support de tube porte-échantillon (29) et [c] un tube flexible (25) ayant une première extrémité en communication fluidique avec ledit porte-aiguille et une deuxième extrémité en communication fluidique avec ledit élément de support (29),
où l'élément de support (29) comprend une partie supérieure cylindrique (55) ayant un raccord (57) à l'intérieur de celle-ci, et ledit raccord (57) comprend une partie d'extrémité (67) apte au raccordement en communication fluidique avec ladite partie tubulaire (15),
**caractérisé en ce que**
[d] ledit porte-aiguille comprend une surface de butée (17) et des moyens de levier aptes à séparer l'aiguille (1) du porte-aiguille, lesdits moyens de levier comprenant un levier (8) ayant une première extrémité (9) apte à être déplacée avec un doigt et une deuxième extrémité (11) disposée entre ladite base de raccordement (5) et ladite surface de butée (17), où ledit porte-aiguille comprend une languette aplatie (27) devant être maintenue entre deux doigts et s'étendant depuis l'un des deux côtés du conduit, où ladite languette aplatie (27) comprend ladite surface de butée (17), et où lesdits moyens de levier sont fixés solidairement audit porte-aiguille,
[e] ledit raccord (57) de l'élément de support (29) a une partie d'extrémité (67) qui est apte à venir en prise à l'intérieur de la partie tubulaire (15),
[f] ledit élément de support (29) comprend au moins deux parois semi-cylindriques (61) s'étendant depuis la partie supérieure cylindrique, séparées mutuellement par des ouvertures latérales (59),
[g] ledit levier (8) comprend une rainure (69), où ladite rainure (69) peut coulisser par friction sur le bord latéral (71) des parois semi-cylindriques (61).

2. Dispositif de prélèvement sanguin selon la revendication 1, **caractérisé en ce qu'**il comprend en outre une aiguille hypodermique (1) ayant une base de raccordement (5), ladite base de raccordement (5) étant apte à être montée sur ladite partie tubulaire (15).

3. Dispositif de prélèvement sanguin selon la revendication 1, **caractérisé en ce que** ladite partie supérieure cylindrique (55) a une pluralité d'ouvertures.

4. Dispositif de prélèvement sanguin selon l'une quelconque des revendications 1 à 3, **caractérisé en ce que** l'élément de support (29) comprend quatre parois semi-cylindriques (61) séparées mutuellement par quatre ouvertures latérales (59).

5. Dispositif de prélèvement sanguin selon l'une quelconque des revendications 1 à 4, **caractérisé en ce qu'**à l'extrémité dudit élément de support (29) un mécanisme de sécurité (74) est raccordé à un bouchon auto-obturant (75) à l'intérieur de celui-ci apte à laisser passer l'air pour remplir ledit dispositif avec une simple pression veineuse.

6. Système pour prendre des échantillons de sang d'un patient, **caractérisé en ce qu'**il comprend :
[i] une pompe pour extraire le sang d'un patient, où ladite pompe est une pompe péristaltique ayant un rotor (31) comprenant au moins une surface de pompage mobile (33) et une surface de pompage fixe (35), apte à loger entre elles un tube flexible (25), où la pompe comprend en outre un élément de tête (37) apte à la fixation d'un élément de support de tube porte-échantillon (29), où l'élément de tête (37) comprend une partie de mise en prise supérieure (47), sous la forme d'un U horizontal et d'une partie inférieure (49) ayant une section transversale plus petite que celle de la partie supérieure (47), et
[ii] un dispositif de prélèvement sanguin selon l'une quelconque des revendications 1 à 5, où ladite partie supérieure cylindrique (55) est de la même taille que ladite partie supérieure de mise en prise (47) de l'élément de tête (37), de sorte qu'elle est apte à être logée à l'intérieur de la partie supérieure de mise en prise (47) en l'insérant le long du U horizontal.

7. Système selon la revendication 6, **caractérisé en ce que** l'élément de tête (37) comprend un capteur de présence de tube porte-échantillon, un capteur de niveau de remplissage et/ou un capteur de vitesse de remplissage.

8. Procédé selon l'une des revendications 6 ou 7, **caractérisé en ce que** l'élément de tête comprend des moyens de retenue à ressort (63) pour l'élément de support (29).

9. Système selon l'une quelconque des revendications 6 à 8, **caractérisé en ce qu'**il comprend des moyens de positionnement de la surface fixe (35) par rapport à la surface mobile (33) aptes à déplacer la surface fixe (35) entre une position espacée et une position de pompage, où les moyens de positionnement comprennent des moyens d'encliquetage réversibles aptes à fixer de manière réversible la surface fixe (35) par rapport à la surface de pompage, et des moyens à ressort poussant ladite surface fixe (35) vers la position espacée.

10. Système selon l'une quelconque des revendications 6 à 9, **caractérisé en ce qu'**il comprend des moyens de commande électronique et un écran de visualisation des données.

11. Système selon l'une quelconque des revendications 6 à 10, **caractérisé en ce qu'**à l'extrémité dudit élément de support (29) un mécanisme de sécurité (74) est raccordé à un bouchon auto-obturant (75) à l'intérieur de celui-ci apte à laisser passer l'air pour remplir ledit dispositif avec une simple pression veineuse.
